# EUROPEAN PATENT SPECIFICATION

(11) **EP 0 956 354 B1**
(45) Date of publication and mention of the grant of the patent: **14.01.2009**
(21) Application number: 97933506.4
(22) Date of filing: 03.07.1997
(51) Int. Cl.: C12N 15/57, C12N 15/09, C12N 1/21, C12N 9/64

(54) **RECOMBINANT N-PROTEINASE, AND THE PRODUCTION, METHODS AND USES THEREOF**
REKOMBINANTE N-PROTEINASE, DEREN HERSTELLUNG, METHODEN UND ANWENDUNGEN
N-PROTEINASE DE RECOMBINAISON, ET PRODUCTION, PROCEDES ET UTILISATIONS DE CETTE DERNIERE

(30) Priority: 03.07.1996 US 21203 P; 02.07.1997 US 886333
(43) Date of publication of application: 17.11.1999
(73) Proprietor: Université de Liège, 4000 Liège (BE)
(72) Inventor: Prockop, Darwin, Philadelphia, PA 19106 (US); Colige, Alain, 5100 Dave (BE); Lapiere, Charles, / (BE)
(74) Representative: Hallybone, Huw George
(86) International application number: PCT/US1997/012427
(87) International publication number: WO 1998/000555

(56) References cited:
- PROC. NATL. ACAD. SCI. U.S.A., March 1997, Vol. 94, COLIGE A, "cDNA Cloning and Expression of Bovine Procollagen I N-Proteinase: A New Member of the Superfamily of Zinc-Metalloproteinases with Binding Sites for Cells and Other Matrix Components", pages 2374-2379, XP002945369
- HALILA R. et al., "Type III Procollagen N-Proteinase: Isolation of a Candidate cDNA Clone and Assignment of the Corresponding Gene to Human Chromosome 16 Matrix", January 1993, Vol. 13, No. 1, pages 9-10, XP002946706
- J. BIOL. CHEM., 14 July 1994, Vol. 270, No. 28, COLIGE A. et al., "Characterization and Partial Amino Acid Sequencing of a 107-kDa Procollagen I N-Proteinase Purified by Affinity Chromatography on Immobilized Type XIV Collagen", pages 16724-16730, XP002945370
- SCOTT,I.C. ET AL.: "Molecular cloning, expression and chromosomal localization of a human gene encoding a 33 kDa putative metallopeptidase (PRSM1)" GENE, vol. 174, 1996, pages 135-143,

## Description

The information disclosed in this Specification was made in part with Government grant support, awarded by the National Institute of Health. The government may have certain rights in the invention disclosed in this Specification.

### I. FIELD OF THE INVENTION

This invention relates generally to the field of N-proteinase and the production, uses and methods thereof.

### II. BACKGROUND OF THE INVENTION

***The ExtraCellular Matrix.*** The most abundant component of the extracellular matrix is collagen. Collagen molecules are generally the result of the trimeric assembly of three polypeptide chains containing, in their primary sequence, (-Gly-X-Y-)ₙ repeats which allow for the formation of triple helical domains. Van der Rest et al. 1991, FASEB J. 5:2814-2823.

During their biosynthesis, the fibrillar collagens, including collagen types I, II and III, are synthesized as precursors, known as procollagens. These procollagens are comprised of a central triple-helical collagen domain extended by propeptides both at the molecules' carboxyl and amino ends. These propeptides, designated as C-propeptide (for the propeptide found at the carboxyl terminal end of procollagen) and N-propeptide (for the propeptide found at the amino terminal end of procollagen), are cleaved during post-translational events by the enzymes C-proteinase and N-proteinase, respectively.

***Diseases Associated With The Abnormal Production of Collagen.*** An array of critical diseases has been associated with the inappropriate or unregulated production of collagen, including pathological fibrosis or scarring, including endocardial sclerosis, idiopathic interstitial fibrosis, interstitial pulmonary fibrosis, perimuscular fibrosis, Symmers' fibrosis, pericentral fibrosis, hepatitis, dermatofibroma, billary cirrhosis, alcoholic cirrhosis, acute pulmonary fibrosis, idiopathic pulmonary fibrosis, acute respiratory distress syndrome, kidney fibrosis/glomerulonephritis, kidney fibrosis/diabetic nephropathy, scleroderma/systemic, scleroderma/local, keloids, hypertrophic scars, severe joint adhesions/arthritis, myelofibrosis, corneal scarring, cystic fibrosis, muscular dystrophy (duchenne's), cardiac fibrosis, muscular fibrosis/retinal separation, esophageal stricture, payronles disease. Further fibrotic disorders may be induced or initiated by surgery, including scar revision/plastic surgeries, glaucoma, cataract fibrosis, corneal scarring, joint adhesions, graft vs. host disease, tendon surgery, nerve entrapment, dupuytren's contracture, OB/GYN adhesions/fibrosis, pelvic adhesions, peridural fibrosis, restenosis. One strategy for the treatment of these diseases is the inhibition of the pathological overproduction of collagen. The identification and isolation of enzymes involved in the collagen production and processing are therefore of major medical interest to provide for suitable targets for drug development.

Similarly, a strategy for the treatment of diseases resulting from the pathological underproduction of collagen, where the underproduction of collagen is the consequence of improper processing of procollagen, is the administration of C-proteinase.

***N-Proteinase.*** N-proteinase is the post-translational enzyme responsible for cleaving the N-propeptide from the procollagen molecule. Type III N-proteinase is specific to type III procollagen and excises the N-propeptide from type III procollagen only. In contrast, type I N-proteinase acts on both collagen types I and II.

The purification of both type I and type III N-proteinase from natural sources, including chicken embryos, has been previously reported. For example, with respect to type III N-proteinase, the isolation and purification of human enzyme from ascitic fluid and placenta were reported in 1985 and 1986, respectively. *See,* Niemela et al., 1985, Biochem. J. 232:145-150; Halila and Peltonen, 1986, Biochem. J. 239:47-52. The isolation and at least partial purification of type I N-proteinase from chick and bovine sources, have also been reported previously. *See,* Kohn et al., 1974, Proc. Natl. Acad. Sci. USA 71:44; Tuderman and Prockop, 1982, Eur. J. Biochem. 125:545-549; Tazawa et al., 1985, J. Biol. Chem. 260:1120-1126; Hojima et al., 1994, J. Biol. Chem. 269:11381-11390; Colige et al., 1995, J. Biol. Chem. 270:16724-16730.

The kinetics of purified naturally-occurring N-proteinases, both Types I and III, have also been studied. Dombrowski and Prockop, 1988, J. Biol. Chem. 263:16545-16552. Prior to the present invention, however, the nucleotide sequence of N-proteinase had not been determined and thus the means for producing recombinant N-proteinase was unknown.

### III. SUMMARY OF THE INVENTION

N-proteinase exists in two forms, a "short" form comprising a molecule approximately 70 kDa in length and a "long" form comprising a molecule approximately 130 kDa in length. The present invention is directed to polynucleotide sequences encoding both the short and long forms of N-proteinase, including fragments of both forms of N-proteinase having the ability to cleave N-propeptide from procollagen.

The present invention is further directed to synthesized or recombinant compositions corresponding to or derived from the polynucleotide sequences of the present invention. In one embodiment of the present invention, the composition is radiolabelled for use in assays.

The present invention is also related to the synthesis of recombinant production of N-proteinase and related compositions. Where N-proteinase is produced recombinantly, the use of a variety of recombinant expressions systems is contemplated, including yeast, plant cell, insect cell, mammalian cell and *E. coli* expression systems.

### IV. DEFINITIONS

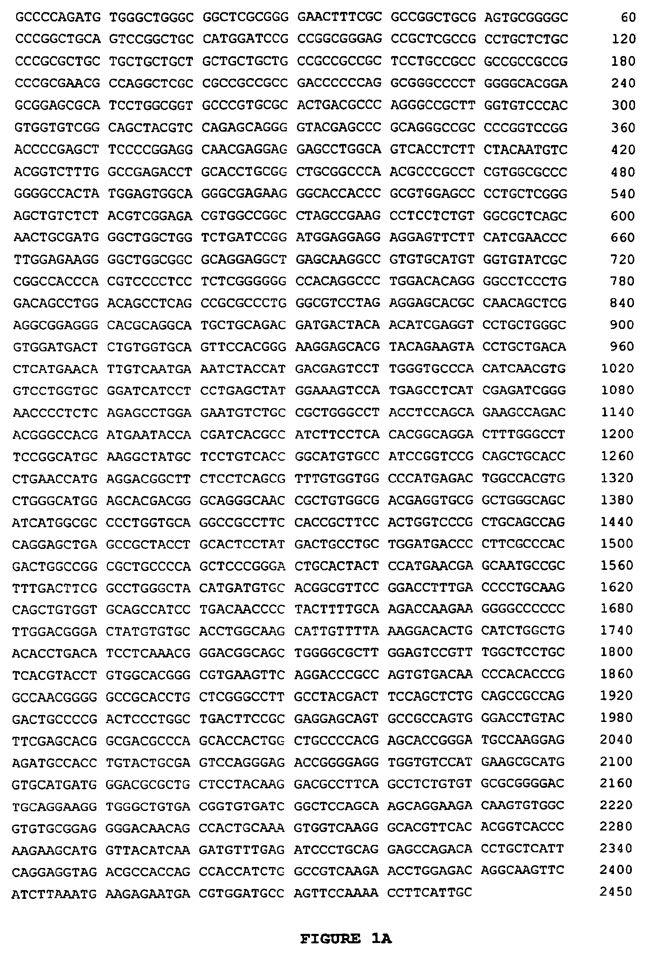

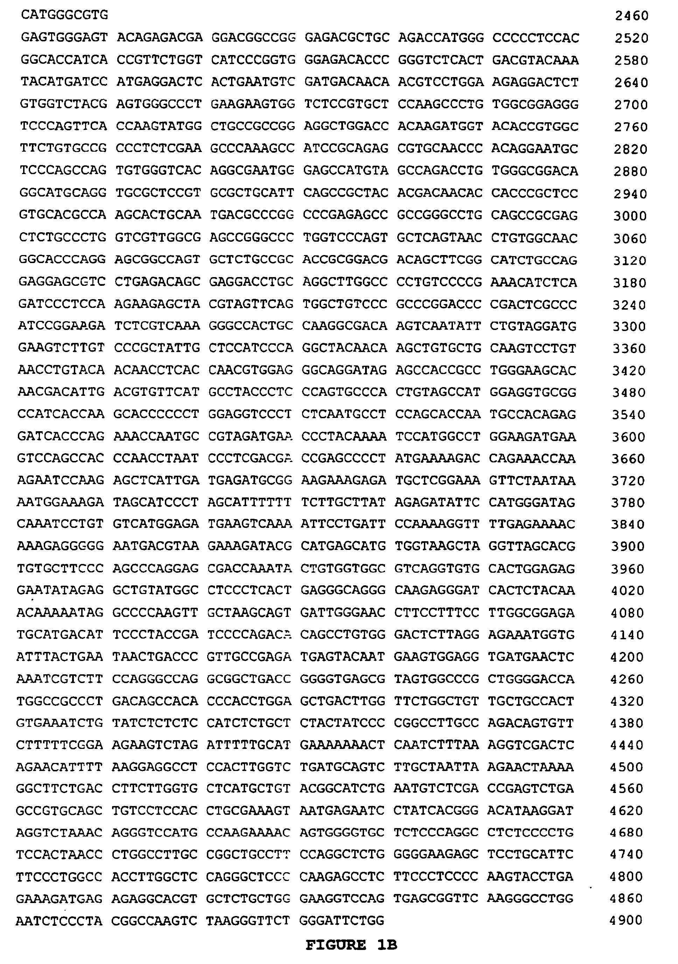

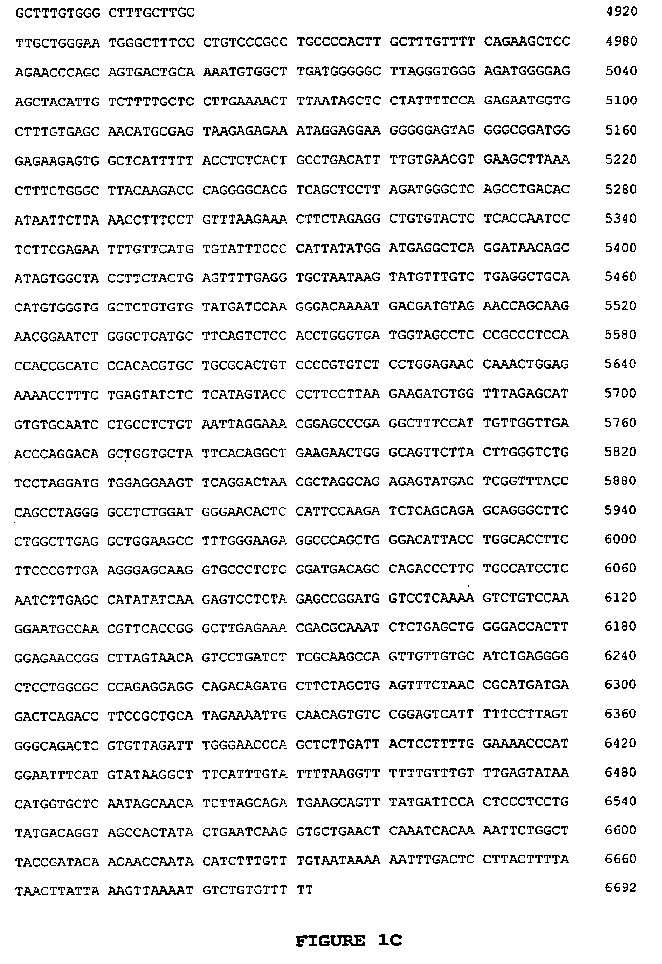

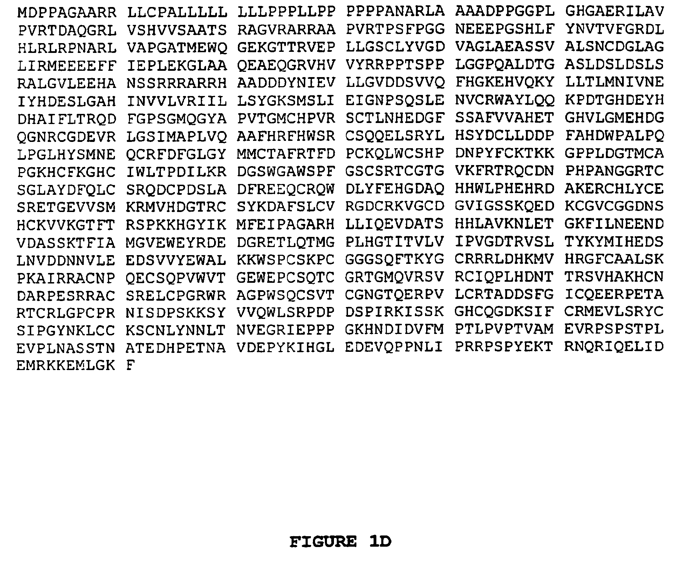

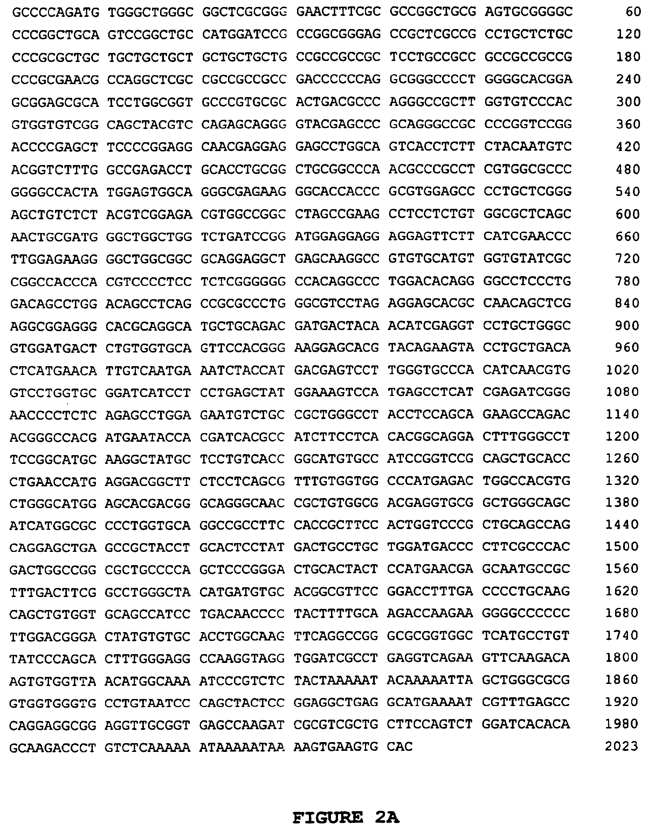

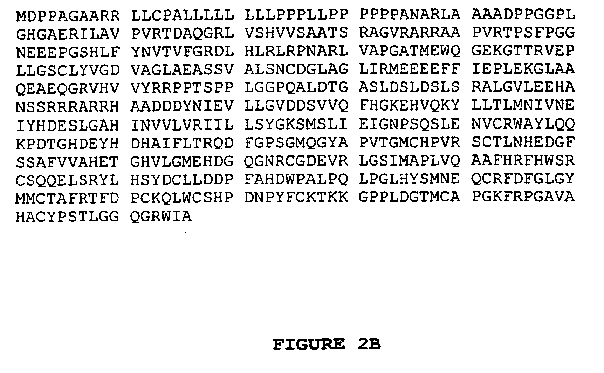

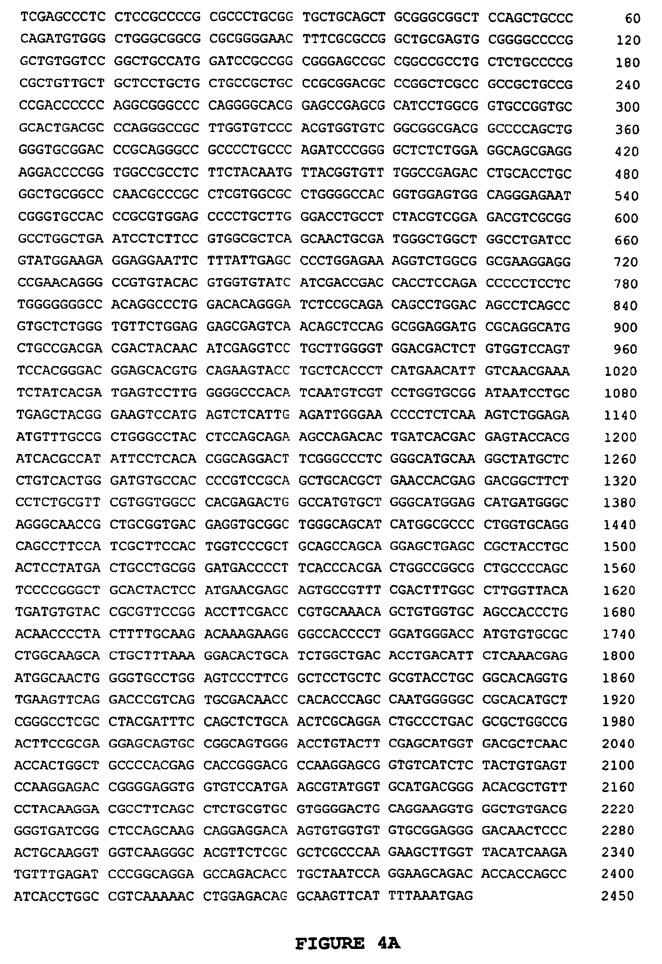

As used in this Specification, the term *"N-Proteinase"* shall mean: (1) a protein encoded by the amino acid sequence as set forth at Figure 1D deduced from the nucleic acid sequences set forth at Figures 1A-1C, a protein encoded by the amino acid sequences as set forth at Figure 2B deduced from the nucleic acid sequence set forth at Figure 2A, and the amino acid sequence encoded from the nucleic acid sequences set fort at Figures 4A-4B; (2) a protein having N-proteinase activity wherein such protein is encoded by the amino acid sequences deduced from the nucleic acid sequences set forth at Figures 1A-1C, Figure 2A, and Figures 4A-4B, wherein one or more amino acids have been added, deleted, mutated, substituted or otherwise altered ("derivative") and the nucleotide sequence encoding said protein can hybridize to the nucleic acid sequence of Figures 1A-1C, Figure 2A and Figures 4A-4B under stringent hybridization conditions; (3) a fragment of N-proteinase or a derivative thereof; and (4) the protein encoded by a naturally-occurring allele or homolog of the gene corresponding to the nucleic acid sequences set forth at Figures 1A-1C, Figure 2A, or Figures 4A-4B.

As used in this Specification, the term *"**Polynucleotide**"* denotes DNA, cDNA and/or RNA, including genomic DNA and mRNA.

As used in this Specification, the phrase ***"Stringent Hybridization*** ***Conditions"*** refers to those hybridizing conditions that (1) employ low ionic strength and high temperature for washing, for example, 0.015 M NaCl/0.0015 M sodium citrate/0.1% SDS at 50°C; (2) employ during hybridization a denaturing agent such as formamide, for example, 50% (vol/vol) formamide with 0.1 % bovine serum albumin/0.1% Ficoll/0.1% polyvinylpyrrolidone/50 mM sodium phosphate buffer at pH 6.5 with 750 mM NaCl, 75 mM sodium citrate at 42 °C; or (3) employ 50% formamide, 5x SSC (0.75 M NaCl, 0.075 M Sodium pyrophosphate, 5x Denhardt's solution, sonicated salmon sperm DNA (50 g/ml), 0.1 % SDS, and 10% dextran sulfate at 42°C, with washes at 42°C in 0.2 x SSC and 0.1 % SDS.

As used in this Specification, the phrase ***"Recombinant Expression Vector"*** refers to a plasmid, virus or other vehicle known in the art that has been manipulated by insertion or incorporation of the N-proteinase sequences.

### V. BRIEF DESCRIPTION OF THE DRAWINGS

FIGURE 1A sets forth a nucleic acid sequence, positions 1 to 2450, of the 130 kDa ("long") form of human N-proteinase.
FIGURE 1B sets forth a nucleic acid sequence, positions 2451 to 4900, of the long form of human N-proteinase.
FIGURE 1C sets forth a nucleic acid sequence, positions 4901 to 6692, of the long form of human N-proteinase.
FIGURE 1D sets forth the amino acid sequence of the long form of human N-proteinase.
FIGURE 2A sets forth a nucleic acid sequence of the 70 kDa ("short") form of human N-proteinase.
FIGURE 2B sets forth the amino acid sequence of the short form of human N-proteinase.
FIGURE 3 sets forth a schematic comparison of the structure of C-proteinase and N-proteinase and identifies a predicted site for cleavage of the proteinases from their inactive to active forms.
FIGURE 4A sets forth a nucleic acid sequence, positions 1 to 2450 of a form of bovine N-proteinase.
FIGURE 4B sets forth a nucleic acid sequence, positions 2451 to 4580 of a form of bovine N-proteinase.
FIGURE 5 sets forth the deduced amino acid sequence of the bovine N-proteinase set forth in FIGURES 4A and 4B.
FIGURE 6 sets forth the oligonucleotide probes used to isolate the nucleic acid molecules encoding bovine N-proteinase.

### VI. DETAILED DESCRIPTION OF THE INVENTION

### A. Polynucleotide Sequence Encoding N-proteinase

***Nucleic Acid Sequence Encoding N-Proteinase.*** The N-proteinase enzyme may be isolated according to the procedures described in Hojima et al., 1989, J. Biol. Chem. 264:11336-11345 and Hojima et al., 1994, J. Biol. Chem. 269:11381-11390. In one preferred embodiment of the invention, N-proteinase may be further purified by use of monoclonal antibodies obtained by injecting mice with the enzyme purified according to the procedures described in Colige et al., 1995, J. Biol. Chem. 270: 16724-16730.

The enzyme is amino terminally blocked such that the amino acid corresponding to N-proteinase cannot be determined using a commercially available apparatus. Thus, in one preferred embodiment, the amino acid sequence comprising N-proteinase may be determined by: (1) digesting the enzyme with endoproteinase LysC; (2) resolving the resulting internal peptides by reversed-phase chromatography; and (3) sequencing the material in each of the resultant peaks.

Nucleic acid probes can then be prepared using the determined amino acid sequences for the N-proteinase peptide fragments. Such probes may be synthesized synthetically and labeled. Preparation techniques for such probes and others are generally set forth in Sambrook et al., 1990, Molecular Cloning, A Laboratory Manual 2d, Ed. , Cold Springs Harbor Laboratory Press, New York, at Chapters 10-11. The nucleic acid probes may be sequenced using any one of the techniques generally described in Sambrook *et al., supra,* at Chapter 13. These nucleic acid probes may be used then to screen a wide array of libraries to isolate and then characterize the full length nucleic acid sequence encoding N-proteinase. For example, the probes may be used to screen a bacteriophage cDNA library or other cDNA library, including libraries constructed using a mammalian expression vector such as pcDNA1 and a genomic library.

The gene encoding N-proteinase may also be isolated by performing a polymerase chain reaction (PCR) using one or more degenerate oligonucleotide primer pools that are designed based on the deduced nucleotide sequence of N-proteinase. The techniques used to identify the nucleic acid sequence of a protein using PCR are described in, for example, Sambrook *et al., supra,* at Chapter 14.

Nucleic acid sequences encoding N-proteinase have been determined and are set forth at Figures 1A-1C (human), Figure 2A (human) and 4A-4B (bovine). It is contemplated that the polynucleotide sequences of the present invention include the sequences set forth in Figures 1A-1C, Figure 2A and Figures 4A-4B, as well as sequences corresponding to the naturally-occurring alleles and homologs to the disclosed sequence, and variants which are the result of polymorphism.

***Other Nucleic Acid Sequences Encoding N-Proteinase.*** In accordance with the invention, nucleotide sequences encoding N-proteinase or functional equivalents thereof may be used to generate recombinant DNA molecules that direct the expression of the protein or a functional equivalent thereof, in appropriate host cells. Alternatively, nucleotide sequences which hybridize, under stringent hybridization conditions, to portions of the N-proteinase sequence may also be used in nucleic acid hybridization assays, Southern and Northern blot analyses, etc. In yet another method, DNA molecules encoding N-proteinase may be isolated by hybridization procedures comprising antibody screening of expression libraries to detect shared structural features.

Due to the inherent degeneracy of the genetic code, other DNA sequences which encode substantially the same or a functionally equivalent amino acid sequence, may be isolated and used in the practice of the invention for the cloning and expression of N-proteinase. Such DNA sequences include those which are capable of hybridizing to the human or bovine N-proteinase sequence under stringent conditions.

Altered DNA sequences which may be used in accordance with the invention include deletions, additions or substitutions of different nucleotide residues resulting in a sequence that encodes the same or a functionally equivalent gene product. The gene product itself may contain deletions, additions or substitutions of amino acid residues within the N-proteinase sequence, which result in a silent change thus producing a functionally equivalent protein. Such amino acid substitutions may be made on the basis of similarity in polarity, charge, solubility, hydrophobicity, hydrophilicity, and/or the amphipatic nature of the residues involved. For example, negatively charged amino acids include aspartic acid and glutamic acid; positively charged amino acids include lysine and arginine; amino acids with uncharged polar head groups having similar hydrophilicity values include the following: leucine, isoleucine, valine; glycine, aniline; asparagine, glutamine; serine, threonine; phenylalanine, tyrosine.

The DNA sequences of the invention may be engineered in order to alter the protein's sequence for a variety of ends including but not limited to alterations which modify processing and expression of the gene product. For example, mutations may be introduced using techniques which are well known in the art, *e.g.*, site-directed mutagenesis to, for example, insert new restriction sites. For example, in certain expression systems such as yeast, host cells may over-glycosylate the gene product. When using such expression systems it may be preferable to alter N-proteinase coding sequence to eliminate any N-linked glycosylation site.

The N-proteinase sequence may be ligated to a heterologous sequence to encode a fusion protein. For example, a fusion protein may be engineered to contain a cleavage site located between the N-proteinase sequence and a heterologous protein sequence, so that N-proteinase can be cleaved away from the heterologous moiety.

The coding sequence of N-proteinase may also be synthesized in whole or in part, using chemical methods well known in the art. *See,* for example, Caruthers et al., 1980, Nucleic Acids Res. Symp. Ser. 7:215-233; Crea and Horn, 1980, Nucleic Acids Res. 9:2331; Matteucci and Caruthers, 1980, Tetrahedron Letters 21:719; and Chow and Kempe, 1981, Nucleic Acids Res. 9:2807-2817. Alternatively, the protein itself could be produced using chemical methods to synthesize the N-proteinase amino acid sequence in whole or in part. For example, peptides can be synthesized by solid phase techniques, cleaved from the resin, and purified by preparative high performance liquid chromatography. *See, e.g.,* Creighton, 1983, Proteins Structures And Molecular Principles, W.H. Freeman and Co., N.Y. pp. 50-60. The composition of the synthetic peptides may be confirmed by amino acid analysis or sequencing. *See, e.g.,* for the Edman degradation procedure, *see,* Creighton, 1983, Proteins, Structures and Molecular Principles, W.H. Freeman and Co., N.Y., pp. 34-49.

### B. Uses Of The N-Proteinase Coding Sequence

The N-proteinase coding sequence may be used for diagnostic purposes for detection of N-proteinase expression. Included in the scope of the invention are oligoribonucleotide sequences, that include antisense RNA and DNA molecules and ribozymes that function to inhibit translation of N-proteinase. Antisense techniques are known in the art and may be applied herein.

Ribozymes are enzymatic RNA molecules capable of catalyzing the specific cleavage of RNA. The mechanism of ribozyme action involves sequence specific hybridization of the ribozyme molecule to complementary target RNA, followed by a endonucleolytic cleavage. Within the scope of the invention are engineered hammerhead motif ribozyme molecules that specifically and efficiently catalyze endonucleolytic cleavage of N-proteinase RNA sequences.

Specific ribozyme cleavage sites within any potential RNA target are initially identified by scanning the target molecule for ribozyme cleavage sites which include the following sequences, GUA, GUU and GUC. Once identified, short RNA sequences of between fifteen (15) and twenty (20) ribonucleotides corresponding to the region of the target gene containing the cleavage site may be evaluated for predicted structural features such as secondary structure that may render the oligonucleotide sequence unsuitable. The suitability of candidate targets may also be evaluated by testing their accessibility to hybridization with complementary oligonucleotides, using ribonuclease protection assays.

Both antisense RNA and DNA molecules and ribozymes of the invention may be prepared by any method known in the art for the synthesis of RNA molecules. These include techniques for chemically synthesizing oligodeoxyribonucleotides well known in the art such as for example solid phase phosphoramidite chemical synthesis. Alternatively, RNA molecules may be generated by *in vitro* and *in vivo* transcription of DNA sequences encoding the antisense RNA molecule. Such DNA sequences may be incorporated into a wide variety of vectors which incorporate suitable RNA polymerase promoters such as the T7 or SP6 polymerase promoters. Alternatively, antisense cDNA constructs that synthesize antisense RNA constitutively or inducibly, depending on the promoter used, can be introduced stably into cell lines.

In addition, mutated forms of N-proteinase, having a dominant negative effect, may be expressed in targeted cell populations to inhibit the activity of endogenously expressed wild-type N-proteinase.

Additionally, the DNA encoding N-proteinase may also have a number of uses for the diagnosis of diseases resulting from aberrant expression of the enzyme. For example, the N-proteinase DNA sequence may be used in hybridization assays of biopsies or autopsies to diagnose abnormalities of expression (*e.g*., Southern or Northern blot analysis, *in situ* hybridization assays).

The N-proteinase cDNA may be used also as a probe to detect the expression of the N-proteinase mRNA.

In addition, the expression of N-proteinase during embryonic development may also be determined using nucleic acid encoding N-proteinase. As addressed, *infra,* insufficient production of N-proteinase is the cause of various disease states, including the Ehlers-Danlos disease. *See, Section VI.H.. In situ* hybridizations using N-proteinase as a probe may be employed to predict *in utero* problems related to such connective tissue diseases. Further, as indicated, *infra,* administration of human N-proteinase, recombinantly produced as described herein, may be used to treat disease states related to insufficient production of N-proteinase. Alternatively, gene therapy approaches may be employed to remedy deficiencies of functional N-proteinase.

Various modifications to the DNA molecules may be introduced as a means of increasing intracellular stability and half-life. Possible modifications include but are not limited to the addition of flanking sequences of ribo- or deoxynucleotides to the 5' and/or 3' ends of the molecule or the use of phosphorothioate or 2' O-methyl rather than phosphodiesterase linkages within the oligodeoxyribonucleotide backbone.

### C. Methods For Making N-Proteinase

***Expression Of N-Proteinase.*** In order to express a biologically active N-proteinase, the nucleotide sequence coding for the protein, or a functional equivalent as described above, *supra,* was inserted into an appropriate expression vector, *i.e*., a vector which contains the necessary elements for the transcription and translation of the inserted coding sequence.

More specifically, methods which are well known to those skilled in the art can be used to construct expression vectors containing the N-proteinase sequence and appropriate transcriptional/translational control signals. These methods include *in vitro* recombinant DNA techniques, synthetic techniques and *in vivo* recombination/genetic recombination. *See, e.g.,* the techniques described in Sambrook et al., 1990, Molecular Cloning: A Laboratory Manual, Cold Spring Harbor Laboratory, N. Y. ; and Ausubel et al., 1989, Current Protocols in Molecular Biology, Greene Publishing Associates and Wiley Interscience, N.Y.

A variety of host-expression vector systems may be utilized to express the N-proteinase coding sequence. These include but are not limited to microorganisms such as bacteria transformed with recombinant bacteriophage DNA, plasmid DNA or cosmid DNA expression vectors containing the N-proteinase coding sequence; yeast, including *Pichia pastoris* and *Hansenula polymorpha,* transformed with recombinant yeast expression vectors containing the N-proteinase coding sequence; insect cell systems infected with recombinant virus expression vectors (*e*.*g*. , baculovirus) containing the N-proteinase coding sequence; plant cell systems infected with recombinant virus expression vectors (*e.g*., cauliflower mosaic virus, CaMV; tobacco mosaic virus, TMV) or transformer with recombinant plasmid expression vectors *(e.g.,* Ti plasmid) containing the N-proteinase coding sequence; or animal cell systems infected with recombinant virus expression vectors (*e.g*., adenovirus, vaccinia virus, human tumor cells (including HT-1080)) including cell lines engineered to contain multiple copies of the N-proteinase DNA either stably amplified (CHO/dhfr) or unstably amplified in double-minute chromosomes (*e*.*g*., murine cell lines). As used herein, it is understood that the term "host-expression vector systems" and more generally, the term "host cells" includes any progeny of the host cell or host-expression vector system. It is further understood that although all progeny may not be identical to the parental cell, as mutations may occur during replication, such progeny are included in the scope of the invention.

The expression elements of these systems vary in their strength and specificities. Depending on the host/vector system utilized, any of a number of suitable transcription and translation elements, including constitutive and inducible promoters, may be used in the expression vector. For example, when cloning in bacterial systems, inducible promoters such as pL of bacteriophage 8, plac, ptrp, ptac (ptrp-lac hybrid promoter) and the like may be used; when cloning in insect cell systems, promoters such as the baculovirus polyhedron promoter may be used; when cloning in plant cell systems, promoters derived from the genome of plant cells (*e.g*., heat shock promoters; the promoter for the small subunit of RUBISCO; the promoter for the chlorophyll a/b binding protein) or from plant viruses (*e.g*., the ³⁵S RNA promoter of CaMV; the coat protein promoter of TMV) may be used; when cloning in mammalian cell systems, promoters derived from the genome of mammalian cells (*e.g*., metallothionein promoter) or from mammalian viruses (*e*.*g*., the adenovirus late promoter; the vaccinia virus 7.5 K promoter) may be used; when generating cell lines that contain multiple copies of the N-proteinase DNA SV40-, BPV- and EBV-based vectors may be used with an appropriate selectable marker.

In bacterial systems, a number of expression vectors may be advantageously selected depending upon the use intended for the expressed N-proteinase. For example, a suitable vector for expression in bacteria includes the T7-based vector as described in Rosenberg et al., 1987, Gene 56:125. As further example, when large quantities of N-proteinase are to be produced to screen peptide libraries, vectors which direct the expression of high levels of protein products that are readily purified may be desirable. Such vectors include but are not limited to the *E. coli* expression vector pUR278 (Ruther et al., 1983, EMBO J. 2:1791), in which the N-proteinase coding sequence may be ligated into the vector in frame with the lac Z coding region so that a hybrid AS-lac Z protein is produced; pIN vectors (Inouye and Inouye, 1985, Nucleic Acids Res. 13:3101-3109; Van Heeke and Schuster, 1989, J. Biol. Chem. 264:5503-5509); and the like. pGEX vectors may also be used to express foreign polypeptides such as N-proteinase with glutathione S-transferase (GST). In general, such fusion proteins are soluble and can easily be purified from lysed cells by adsorption to glutathione-agarose beads followed by elution in the presence of free glutathione. The pGEX vectors are designed to include thrombin or factor Xa protease cleavage sites so that the cloned polypeptide of interest can be released from the GST moiety.

More generally, where the host is a procaryote, competent cells which are capable of DNA uptake can be prepared from cells harvested after exponential growth and subsequently treated by the CaCl₂, or alternatively MgCl₂ or RbCl, method using procedures well known in the art.

Where the host cell is a eukaryote, various methods of DNA transfer can be used. These include transfection of DNA by calcium phosphate-precipitates, conventional mechanical procedures, including microinjection, insertion of a plasmid encased in liposomes, or use of virus vectors. Eukaryotic cells may also be cotransformed with DNA sequences encoding the polypeptide of the invention, and a second foreign DNA molecule encoding a selectable phenotype, such as herpes simplex thymidine kinase gene. Another method is to use a eukaryotic viral vector, such as *Simian Virus* 40 (SV40) or bovine papilloma virus, to transiently infect or transform eukaryotic cells and express protein. *See,* Eukaryotic Viral Vectors, 1992, Cold Spring Harbor Laboratory, Gluzman, Ed.). Eukaryotic host cells include yeast, mammalian cells, insect cells and plant cells.

In yeast, a number of vectors containing constitutive or inducible promoters may be used. For a review, *see,* Current Protocols in Molecular Biology, Vol. 2, 1988, Ausubel et al., Ed., Greene Publish. Assoc. and Wiley Interscience, Ch. 13; Grant et al., 1987, Methods in Enzymology, Wu and Grossman, Eds., Acad. Press, N.Y., 153:516-544; Glover, 1986, DNA Cloning, Vol. II, IRL Press, Wash., D.C., Ch. 3; Bitter, 1987, Heterologous Gene Expression in Yeast, Methods in Enzymology, Berger and Kimmel, Eds., Acad. Press, N.Y., 152:673-684; and The Molecular Biology of the Yeast Saccharomyces, 1982, Strathern et al., Eds., Cold Spring Harbor Press, Vols. I and II. For example, various shuttle vectors for the expression of foreign genes in yeast have been reported. Heinemann et al., 1989, Nature 340:205; Rose et al., 1987, Gene 60:237.

In cases where plant expression vectors are used, the expression of the N-proteinase coding sequence may be driven by any of a number of promoters. For example, viral promoters such as the 35S RNA and 19S RNA promoters of CaMV (Brisson et al., 1984, Nature 310:511-514), or the coat protein promoter of TMV (Takamatsu et al., 1987, EMBO J. 6:307-311) may be used; alternatively, plant promoters such as the small subunit of RUBISCO (Coruzzi et al., 1984, EMBO J. 3:1671-1680; Broglie et al., 1984, Science 224:838-843); or heat shock promoters, *e.g*., soybean hsp17.5-E or hsp17.3-B (Gurley et al., 1986, Mol. Cell. Biol. 6:559-565) may be used. These constructs can be introduced into plant cells using Ti plasmids, Ri plasmids, plant virus vectors, direct DNA transformation, microinjection, electroporation, etc. For reviews of such techniques, *see, e.g.,* Weissbach and Weissbach, 1988, Methods for Plant Molecular Biology, Academic Press, NY, Section VIII, pp. 421-463; Grierson and Corey, 1988, Plant Molecular Biology, 2d Ed., Blackie, London, Ch. 7-9.

In an insect system, an alternative expression system could be used to express N-proteinase. In one such system, Baculovirus is used as a vector to express foreign genes. The virus then grows in the insect cells. The N-proteinase coding sequence may be cloned into non-essential regions (for example the polyhedron gene) of the virus and placed under control of a Baculovirus promoter. These recombinant viruses are then used to infect insect cells in which the inserted gene is expressed. *See, e.g.,* Smith et al., 1983, J. Virol. 46:584; Smith, U.S. Patent No. 4,215,051.

In mammalian host cells, a number of viral based expression systems may be utilized. In cases where an adenovirus is used as an expression vector, the N-proteinase coding sequence may be ligated to an adenovirus transcription/translation control complex, *e.g*., the late promoter and tripartite leader sequence. This chimeric gene may then be inserted in the adenovirus genome by *in vitro* or *in vivo* recombination. Insertion in a non-essential region of the viral genome (*e.g*., region E1 or E3) will result in a recombinant virus that is viable and capable of expressing N-proteinase in infected hosts. *See, e.g.,* Logan and Shenk, 1984, Proc. Natl. Acad. Sci. USA 81:3655-3659. Alternatively, the vaccinia 7.5K promoter may be used. *See, e.g.,* Mackett et al., 1982, Proc. Natl. Acad. Sci. USA 79:7415-7419; Mackett et al., 1984, J. Virol. 49:857-864; Panicali et al., 1982, Proc. Natl. Acad. Sci. USA 79:4927-4931. Preferably, the vehicle used is a *Forest Semiliki Virus.*

In another embodiment, the N-proteinase sequence is expressed in human tumor cells, such as HT-1080, which have been stably transfected with calcium phosphate precipitation and a neomycin resistance gene. In yet another embodiment, the pMSXND expression vector or the like is used for expression in a variety of mammalian cells, including COS, BHK, 293 and CHO cells. Lee and Nathans, 1988, J. Biol. Chem. 263:3521.

Specific initiation signals may also be required for efficient translation of inserted N-proteinase coding sequences. These signals include the ATG initiation codon and adjacent sequences. In cases where the entire N-proteinase gene, including its own initiation codon and adjacent sequences, is inserted into the appropriate expression vector, no additional translational control signals may be needed. However, in cases where only a portion of the N-proteinase coding sequence is inserted, exogenous translational control signals, including the ATG initiation codon, must be provided. Furthermore, the initiation codon must be in phase with the reading frame of the N-proteinase coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. *See, e.g.,* Bitter et al., 1987, Methods in Enzymol. 153:516-544.

In addition, a host cell strain may be chosen which modulates the expression of the inserted sequences, or modifies and processes the gene product in the specific fashion desired. Such modifications (*e.g*., glycosylation) and processing (*e*.*g*., cleavage) of protein products may be important for the function of the protein. Different host cells have characteristic and specific mechanisms for the post-translational processing and modification of proteins. Appropriate cells lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include but are not limited to CHO, VERO, BHK, HeLa, COS, MDCK, 293, WI38, HT-1080, etc.

For long-term, high-yield production of recombinant proteins, stable expression is preferred. For example, cell lines which stably express N-proteinase may be engineered. Rather than using expression vectors which contain viral origins of replication, host cells can be transformed with N-proteinase DNA controlled by appropriate expression control elements (*e.g*., promoter, enhancer, sequences, transcription terminators, polyadenylation sites, etc.), and a selectable marker. Following the introduction of foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines.

In addition, it is contemplated that N-proteinase can be co-expressed with collagen or other collagen-related enzymes within the same or different host expression systems such that the expressed N-proteinase can act directly only the second protein (e. g. , procollagen).

A number of selection systems may be used, including but not limited to the herpes simplex virus thymidine kinase (Wigler et al., 1977, Cell 11:223), hypoxanthine-guanine phosphoribosyltransferase (Szybalska and Szybalski, 1962, Proc. Natl. Acad. Sci. USA 48:2026), and adenine phosphoribosyltransferase (Lowy et al., 1980, Cell 22:817) genes can be employed in tk, hgprt or aprt cells, respectively.

Also, antimetabolite resistance can be used as the basis of selection for dhfr, which confers resistance to methotrexate (Wigler et al., 1980, Proc. Natl. Acad. Sci. USA 77:3567; O'Hare et al., 1981, Proc. Natl. Acad. Sci. USA 78:1527); gpt, which confers resistance to mycophenolic acid (Mulligan and Berg, 1981, Proc. Natl. Acad. Sci. USA 78:2072); neo, which confers resistance to the aminoglycoside G-418 (Colberre-Garapin et al., 1981, J. Mol. Biol. 150:1); and hygro, which confers resistance to hygromycin (Santerre et al., 1984, Gene 30: 147) genes. Recently, additional selectable genes have been described, namely trpB, which allows cells to utilize indole in place of tryptophan; hisD, which allows cells to utilize histinol in place of histidine (Hartman and Mulligan, 1988, Proc. Natl. Acad. Sci. USA 85:8047), and ODC (ornithine decarboxylase) which confers resistance to the ornithine decarboxylase inhibitor, 2-(difluoromethyl)-DL-ornithine, DFMO (McConlogue, 1987, In: Current Communications in Molecular Biology, Cold Spring Harbor Laboratory).

The isolation and purification of host cell expressed polypeptides of the invention may be by any conventional means such as, for example, preparative chromatographic separations and immunological separations such as those involving the use of monoclonal or polyclonal antibody.

***Identification Of Transfectants Or Transformants That Express N-Proteinase.*** The host cells which contain the coding sequence and which express the biologically active gene product may be identified by at least four general approaches: (a) DNA-DNA or DNA-RNA hybridization; (b) the presence or absence of "marker" gene functions; (c) assessing the level of transcription as measured by the expression of N-proteinase mRNA transcripts in the host cell; and (d) detection of the gene product as measured by an assay or by its biological activity.

In the first approach, the presence of the N-proteinase coding sequence inserted in the expression vector can be detected by DNA-DNA or DNA-RNA hybridization using probes comprising nucleotide sequences that are homologous to the N-proteinase coding sequence, respectively, or portions or derivatives thereof.

In the second approach, the recombinant expression vector/host system can be identified and selected based upon the presence or absence of certain "marker" gene functions (*e.g*., resistance to antibiotics, resistance to methotrexate, transformation phenotype, occlusion body formation in baculovirus, etc.). For example, in a preferred embodiment, the N-proteinase coding sequence is inserted within a neomycin-resistance marker gene sequence of the vector, and recombinants containing the N-proteinase coding sequence can be identified by the absence of the marker gene function. Alternatively, a marker gene can be placed in tandem with the N-proteinase sequence under the control of the same or different promoter used to control the expression of the N-proteinase coding sequence. Expression of the marker in response to induction or selection indicates expression of the N-proteinase coding sequence.

In the third approach, transcriptional activity for the N-proteinase coding region can be assessed by hybridization assays. For example, RNA can be isolated and analyzed by Northern blot using a probe homologous to the N-proteinase coding sequence or particular portions thereof. Alternatively, total nucleic acids of the host cell may be extracted and assayed for hybridization to such probes.

The fourth approach involves the detection of the biologically active or immunologically reactive N-proteinase gene product. A number of assays can be used to detect N-proteinase activity including but not limited to those assays described in U.S. Patent No. 5,408,040.

### D. Structure Of N-Proteinase

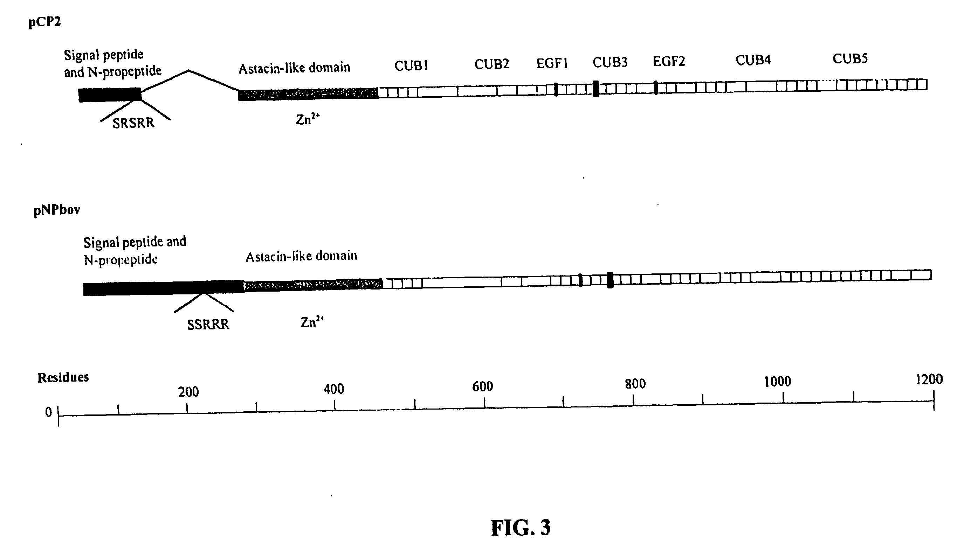

The structural organization of N-proteinase is similar to C-proteinase insofar as the protein is comprised of identifiable domains, which include a large proenzyme domain, an astacin protease-like domain and a large C-terminal region. The structure of N-proteinase, as compared to C-proteinase, is set forth at FIGURE 3. Unlike C-proteinase, N-proteinase does not have repetitive CUB and EGF-like domains; rather, a number of regions comprising N-proteinase contain one to four amino acids which are identical in position and identity to the pCP-2 form of C-proteinase.

### E. Pharmaceutical Formulations And Routes Of Administration

The molecules of the present invention can be administered to a patient in need, alone, or in pharmaceutical compositions where one or more of the molecules are mixed with suitable carriers or excipient(s) at doses to treat or ameliorate a variety of disorders.

Whether the composition is comprised of N-proteinase alone or N-proteinase and additional agents as the active ingredient, such composition is prepared by combining, in a pharmaceutically acceptable carrier substance, *e.g.*, inert gels or liquids, the purified N-proteinase and the other active ingredients.

A therapeutically effective dose further refers to that amount of the compound sufficient to result in amelioration of symptoms. Techniques for formulation and administration of the compounds of the instant application may be found in "Remington's Pharmaceutical Sciences, " Mack Publishing Co. , Easton, PA, latest edition.

### 1. Routes Of Administration

Suitable routes of administration may, for example, include oral, rectal, transmucosal, or intestinal administration; parenteral delivery, including intramuscular, subcutaneous, intramedullary injections, as well as intrathecal, direct intraventricular, intravenous, intraperitoneal, intranasal, or intraocular injections.

Alternately, one may administer the compound in a local rather than systemic manner, for example, via injection of the compound directly into an area requiring N-proteinase, often in a depot or sustained release formulation.

Furthermore, one may administer the drug in a targeted drug delivery system, for example, in a liposome coated with a specific antibody, targeting, for example, cartilage. The liposomes will be targeted to and taken up selectively by the afflicted tissue.

### 2. Composition/Formulation

The pharmaceutical compositions of the present invention may be manufactured in a manner that is itself known, e. g. , by means of conventional mixing, dissolving, granulating, dragee-making, levigating, emulsifying, encapsulating, entrapping or lyophilizing processes.

Pharmaceutical compositions for use in accordance with the present invention thus may be formulated in conventional manner using one or more physiologically acceptable carriers comprising excipients and auxiliaries which facilitate processing of the active molecules into preparations which can be used pharmaceutically. Proper formulation is dependent upon the route of administration chosen.

For injection, the agents of the invention may be formulated in aqueous solutions, preferably in physiologically compatible buffers such as Hanks's solution, Ringer's solution, or physiological saline buffer. For transmucosal administration, penetrants appropriate to the barrier to be permeated are used in the formulation. Such penetrants are generally known in the art.

For oral administration, the compounds can be formulated readily by combining the active compounds with pharmaceutically acceptable carriers well known in the art. Such carriers enable the compounds of the invention to be formulated as tablets, pills, dragees, capsules, liquids, gels, syrups, slurries, suspensions and the like, for oral ingestion by a patient to be treated. Pharmaceutical preparations for oral use can be obtained solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate.

Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. All formulations for oral administration should be in dosages suitable for such administration.

For buccal administration, the compositions may take the form of tablets or lozenges formulated in conventional manner.

For administration by inhalation, the compounds for use according to the present invention are conveniently delivered in the form of an aerosol spray presentation from pressurized packs or a nebuliser, with the use of a suitable propellant, e. g. , dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of, *e.g.,* gelatin for use in an inhaler or insufflator may be formulated containing a powder mix of the compound and a suitable powder base such as lactose or starch.

The molecules may be formulated for parenteral administration by injection, *e.g.*, by bolus injection or continuous infusion. Formulations for injection may be presented in unit dosage form, e. g. , in ampoules or in multi-dose containers, with an added preservative. The compositions may take such forms as suspensions, solutions or emulsions in oily or aqueous vehicles, and may contain formulatory agents such as suspending, stabilizing and/or dispersing agents.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

Alternatively, the active ingredient may be in powder form for constitution with a suitable vehicle, *e.g.*, sterile pyrogen-free water, before use.

The compounds may also be formulated in rectal compositions such as suppositories or retention enemas, e. g. , containing conventional suppository bases such as cocoa butter or other glycerides.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

A pharmaceutical carrier for the hydrophobic molecules of the invention is a cosolvent system comprising benzyl alcohol, a nonpolar surfactant, a water-miscible organic polymer, and an aqueous phase. The cosolvent system may be the VPD co-solvent system. VPD is a solution of 3% w/v benzyl alcohol, 8% w/v of the nonpolar surfactant polysorbate 80, and 65% w/v polyethylene glycol 300, made up to volume in absolute ethanol. The VPD co-solvent system (VPD:5W) consists of VPD diluted 1:1 with a 5% dextrose in water solution. This co-solvent system dissolves hydrophobic compounds well, and itself produces low toxicity upon systemic administration. Naturally, the proportions of a co-solvent system may be varied considerably without destroying its solubility and toxicity characteristics. Furthermore, the identity of the co-solvent components may be varied: for example, other low-toxicity nonpolar surfactants may be used instead of polysorbate 80; the fraction size of polyethylene glycol may be varied; other biocompatible polymers may replace polyethylene glycol, e. g. , polyvinyl pyrrolidone; and other sugars or polysaccharides may substitute for dextrose.

Alternatively, other delivery systems for hydrophobic molecules may be employed. Liposomes and emulsions are well known examples of delivery vehicles or carriers for hydrophobic drugs. Certain organic solvents such as dimethylsulfoxide also may be employed, although usually at the cost of greater toxicity. Additionally, the compounds may be delivered using a sustained-release system, such as semipermeable matrices of solid hydrophobic polymers containing the therapeutic agent. Various of sustained-release materials have been established and are well known by those skilled in the art. Sustained-release capsules may, depending on their chemical nature, release the compounds for a few weeks up to over 100 days. Depending on the chemical nature and the biological stability of the therapeutic reagent, additional strategies for protein stabilization may be employed.

The pharmaceutical compositions also may comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

### 3. Effective Dosage

Pharmaceutical compositions suitable for use in the present invention include compositions wherein the active ingredients are contained in an effective amount to achieve its intended purpose. More specifically, a therapeutically effective amount means an amount effective to prevent development of or to alleviate the existing symptoms of the subject being treated. Determination of the effective amounts is well within the capability of those skilled in the art, especially in light of the detailed disclosure provided herein.

For any compound used in the method of the invention, the therapeutically effective dose can be estimated initially from cell culture assays. For example, a dose can be formulated in animal models to achieve a circulating concentration range that includes the IC₅₀ as determined in cell culture (*i.e*., the concentration of the test compound which achieves a half-maximal N-proteinase activity). Such information can be used to more accurately determine useful doses in humans.

A therapeutically effective dose refers to that amount of the molecule hat results in amelioration of symptoms or a prolongation of survival in a patient. Toxicity and therapeutic efficacy of such molecules can be determined by standard pharmaceutical procedures in cell cultures or experimental animals, *e.g*. , for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio between LD₅₀ and ED₅₀. Molecules which exhibit high therapeutic indices are preferred. The data obtained from these cell culture assays and animal studies can be used in formulating a range of dosage for use in human. The dosage of such molecules lies preferably within a range of circulating concentrations that include the ED₅₀ with little or no toxicity. The dosage may vary within this range depending upon the dosage form employed and the route of administration utilized. The exact formulation, route of administration and dosage can be chosen by the individual physician in view of the patient's condition. *See, e.g. ,* Fingl et al., 1975, in "The Pharmacological Basis of Therapeutics", Ch. 1 p.1.

Dosage amount and interval may be adjusted individually to provide plasma levels of the active moiety which are sufficient to maintain the induction effects of N-proteinase, or minimal effective concentration (MEC). The MEC will vary for each compound but can be estimated from *in vitro* data; for example, the concentration necessary to achieve 50-90% activity of N-proteinase to induce bone growth using the assays described herein. Dosages necessary to achieve the MEC will depend on individual characteristics and route of administration. However, HPLC assays or bioassays can be used to determine plasma concentrations.

Dosage intervals can also be determined using MEC value. Compounds should be administered using a regimen which maintains plasma levels above the MEC for 10-90% of the time, preferably between 30-90% and most preferably between 50-90% .

In cases of local administration or selective uptake, the effective local concentration of the drug may not be related to plasma concentration.

The amount of composition administered will, of course, be dependent on the subject being treated, on the subject's weight, the severity of the affliction, the manner of administration and the judgment of the prescribing physician.

### 4. Packaging

The compositions may, if desired, be presented in a pack or dispenser device which may contain one or more unit dosage forms containing the active ingredient. The pack may, for example, comprise metal or plastic foil, such as a blister pack. The pack or dispenser device may be accompanied by instructions for administration. Compositions comprising a compound of the invention formulated in a compatible pharmaceutical carrier may also be prepared, placed in an appropriate container, and labelled for treatment of an indicated condition. Suitable conditions indicated on the label may include treatment of disorders or diseases in which the administration of a compound of the present invention is desired to ameliorate either the disease or disorder or symptoms related to such disease or disorder.

### F. Assays For Detecting N-Proteinase Activity

Methods for measuring cleavage of N-propeptides by N-proteinase are generally known (for review, *see,* Kadler et al., 1995, Methods Enzymol. 248:756-771). Additionally, a rapid precipitation assay and an electrophoretic assay are useful in detecting and measuring N-proteinase activity, *see,* Nusgens et al., 1979, Anal. Biochem. 95:406-412.

*Rapid Precipitation Assay.* The rapid precipitation assay provides that the reaction products (procollagen and N-proteinase or N-proteinase-like protein) are precipitated with cold ethanol so that the ¹⁴C-labeled N-propeptides are recovered in the supernatant. More specifically, 10 µl of type I procollagen (1.3 µg; 40,000 cpm in 0.1 M Tris-HCl, 0.4 M NaCl, 0.01% NaN₃, pH 7.5) in 1.5 ml polypropylene tubes were incubated with 90 µl of enzyme sample in a the above buffer, pH 7.5, for 1 hour at 35°C. The salt concentration of the reaction mixture was approximately 0.05 M Tris-HCl, 0.15 M NaCl, 5 mM CaCl₂, 0.005% Brij 35, and 0.01% NaN₃, pH 7.5. To stop the reaction 100 µl of 15 mM EDTA in 0.15 M Tris-HCl, 0.3 M NaCl, and 0.01 % NaN₃, pH 7.5 and 100 µl of chilled 81 % ethanol was added. The sample is then vigorously mixed and kept in an ice bath for one (1) hour and then centrifuged at 15,000x g for 15 minutes. The supernatant, approximately 200 µl should be withdrawn, added to 5 ml of an aqueous scintillation fluid and counted for 2 minutes in a liquid scintillation counter. In a preferred method, all samples are assayed in duplicate.

Samples without enzyme and samples in which EDTA was added before the reaction gave values of about 400 cpm. Enzyme activity was proportional to the amount of enzyme added and to the time of incubation over the range of 200 to 900 cpm above background. The rapid assay was used to define enzyme units as 1 unit equal to the amount of enzyme that cleaves 1 µg of type I procollagen in one hour at 35°C under the standard reaction conditions and assuming that the N-propeptides contain about 10% of the ¹⁴C-label.

***Electrophoretic Assay.*** A 50 or 100 µl reaction mixture of N-proteinase and type I procollagen is prepared according to the method set forth in the rapid detection assay and the mixture is then incubated at 35°C for 15 to 180 minutes. The mixture is then mixed with 50 to 100 µl of 0.25 M Tris-HCl, 4% SDS, 15 mM EDTA, 20% glycerol and 0.002% bromphenol blue with or without 4% 2-mercaptoethanol, pH 6.8. The sample is then heated to 100°C for five (5) minutes. Unreduced samples are then separated by electrophoresis on a polyacrylamide slab gel comprised of a 3.5% stacking gel and a 4 to 14% polyacrylamide separation gel. For reduced samples, a stacking gel of 3.5% polyacrylamide and a separation gel of 5.5 or 15% polyacrylamide was used. The gels were analyzed with a phosphor storage plate imager.

### G. Methods For Identifying Inhibitors Of N-Proteinase Activity

Compounds, peptides and antibodies which inhibit the activity of N-proteinase may be determined by use of the polypeptides of the present invention. Specifically, the following assay system for N-proteinase may be used to determine the effectiveness of a compound, peptide or antibody to inhibit N-proteinase:
Samples of 1.3 µg of procollagen and potential N-proteinase inhibitors in an assay buffer (90 µl final volume), as described in Hojima et al., 1994, J. Biol. Chem. 269:11381-11390, are incubated for ten minutes at room temperature, and 2 µl of the purified N-proteinase (0.19 unit) is added. The samples are then incubated for 60 minutes at 35°C in a water bath. The enzyme reaction is stopped by the addition of 25 µl of 5x concentrated sample buffer containing 10% β-mercaptoethanol. After SDS-gel electrophoresis in 6% polyacrylamide gels, gels are dried and analyzed by fluorography after exposure to X-ray films for four hours or by scanning with the phosphor storage plate for the quantitation of N-proteinase activity. The enzyme activity is calculated from the amounts of pCα1 and pCα2 polypeptide chains, assuming that the molecular masses are 135 kDa and 130 kDa, respectively and corrected for uncleaved proα1 and proα2 chains with masses of 155 kDa and 135 kDa, respectively.

### H. Uses Of N-Proteinase Polypeptides

***Production Of Mature Collagen.*** Recombinantly produced N-proteinase may be used for production of mature collagen *in vitro.* For example, a procollagen cDNA may be expressed *in vitro,* and the resulting procollagen processed using recombinantly produced N-proteinase as described herein. Preferably, C-proteinase protein, for example prepared as described in copending U.S. Application Ser. No. 08/609,187, filed March 1, 1996, is further employed to achieve cleavage of the C-terminal C-propeptides.

***Production Of Antibodies To Epitopes Of Recombinantly Produced N-Proteinase.*** Various procedures known in the art may be used for the production of antibodies to epitopes of the recombinantly produced N-proteinase. Such antibodies include but are not limited to polyclonal, monoclonal, chimeric, single chain, Fab fragments and fragments produced by an Fab expression library. Neutralizing antibodies, *i.e*., those which compete for the catalytic domain of the N-proteinase are especially preferred for diagnostics and therapeutics. Such antibodies may be employed, *e.g*. , for the treatment of fibrosis.

Monoclonal antibodies that bind N-proteinase may be radioactively labeled allowing one to follow their location and distribution in the body after injection. Radioactivity tagged antibodies may be used as a non-invasive diagnostic tool for imaging sites of collagen production associated with a number of diseases including fibrosis, and rheumatoid arthritis.

For the production of antibodies, various host animals may be immunized by injection with the N-proteinase protein including but not limited to rabbits, mice, rats, etc. Various adjuvants may be used to increase the immunological response, depending on the host species, including but not limited to Freund's (complete and incomplete), mineral gels such as aluminum hydroxide, surface active substances such as lysolecithin, pluronic polyols, polyanions, peptides, oil emulsions, keyhole limpet hemocyanin, dinitrophenol, and potentially useful human adjuvants such as BCG (bacille Calmette-Guerin) and *Corynebacterium parvum.*

Monoclonal antibodies to N-proteinase may be prepared by using any technique which provides for the production of antibody molecules by continuous cell lines in culture. These include but are not limited to the hybridoma technique originally described by Kohler and Milstein, 1975, Nature 256:495-497, the human B-cell hybridoma technique (Kosbor et al., 1983, Immunology Today 4:72; Cote et al., 1983, Proc. Natl. Acad. Sci. USA 80:2026-2030) and the EBV-hybridoma technique (Cole et al., 1985, Monoclonal Antibodies and Cancer Therapy, Alan R. Liss, Inc., pp. 77-96). In addition, techniques developed for the production of "chimeric antibodies" (Morrison et al., 1984, Proc. Natl. Acad. Sci. USA 81:6851-6855; Neuberger et al., 1984, Nature 312:604-608; Takeda et al., 1985, Nature 314:452-454) by splicing the genes from a mouse antibody molecule of appropriate antigen specificity together with genes from a human antibody molecule of appropriate biological activity can be used. Alternatively, techniques described for the production of single chain antibodies (U.S. Patent 4,946,778) can be adapted to produce N-proteinase-specific single chain antibodies.

Antibody fragments which contain specific binding sites of N-proteinase may be generated by known techniques. For example, such fragments include but are not limited to: the F(ab')₂ fragments which can be produced by pepsin digestion of the antibody molecule and the Fab fragments which can be generated by reducing the disulfide bridges of the F(ab')₂ fragments. Alternatively, Fab expression libraries may be constructed (Huse et al., 1989, Science 246:1275-1281) to allow rapid and easy identification of monoclonal Fab fragments with the desired specificity to N-proteinase.

***Treatment Of Disorders Related To N-Proteinase.*** Various disease states, such as Ehlers-Danlos disease, results from the insufficient production of N-proteinase *in vivo. See,* Nusgens et al., 1992, Nature 1:214-217.
Administration of human N-proteinase to a patient suffering from a disease or disorder caused by the lack of N-proteinase, can therefore ameliorate such disease state.

The below examples explain the invention in more detail. The following preparations and examples are given to enable those skilled in the art to more clearly understand and to practice the present invention. The present invention, however, is not limited in scope by the exemplified embodiments, which are intended as illustrations of single aspects of the invention only, and methods which are functionally equivalent are within the scope of the invention. Indeed, various modifications of the invention in addition to those described herein will become apparent to those skilled in the art from the foregoing description and accompanying drawings. Such modifications are intended to fall within the scope of the appended claims.

### VII. EXAMPLES

### A. Example 1: Characterization Of Polynucleotide Molecule Encoding Bovine N-Proteinase

### 1. Purification Of N-Proteinase

N-proteinase (PCI-NP) was isolated according to the following procedure, wherein such procedure was performed at 4°C or in an ice bath unless otherwise stated:
***Step I: Preparation Of Bovine Skin Extracts.*** Skin was collected from fetal calves at the third trimester stage. 250 g of material was ground at liquid nitrogen temperature and homogenized with an Ultra Turrax (8000 rpm) in 500 ml of washing buffer (50 mM sodium cacodylate, pH 7.5, 0.25 M sucrose, 2 mM CaCl₂, 2.5 mM NEM, 0.5 mM PMSF, and 0.02% NaN₃). After centrifugation (20,000x g for 10 min), the pellet was collected, and the washing procedure was repeated once. Pellets were then suspended in 950 ml of extraction buffer (50 mM sodium cacodylate, pH 7.5, 1 M KCl, 2 mM CaCl₂, 0.02% Brij) supplemented with 1.25 mM NEM and 0.25 mM PMSF. After shaking for 18 h at 4°C, the samples were centrifuged for 10 min at 15,000x g. The supernatants were collected, and extraction of the pellets was repeated once.
***Step II: Ammonium Sulfate Precipitation.*** The proteins in the pooled supernatants were precipitated by adding ammonium sulfate at 40% saturation. The solution was stirred 18 h at 4°C and centrifuged at 15,000x g for 30 min. The precipitate was dissolved in extraction buffer and dialyzed.
***Step III: Affinity Chromatography On Concanavalin A-Sepharose*** The sample was loaded on a 300-ml concanavalin A-Sepharose (Pharmacia LKB Biotechnology, Uppsala, Sweden) column and extensively washed with the extraction buffer. Elution was carried out in the same buffer containing 0.5 M α-methyl-D-mannoside. Active fractions were pooled and dialyzed against buffer H (50 mM sodium cacodylate, pH 7.5, 0.2 M NaCl, 2 M CaCl₂, 0.02% Brij).
***Step IV: Chromatography On Heparin-Sepharose.*** The enzyme preparation from step III was applied to a 75-ml heparin-Sepharose (Pharmacia) column equilibrated in buffer H. After washing, elution was performed with a linear gradient prepared from 250 ml of buffer H and 250 ml of buffer H containing 0.95 M KCl. The most active fractions, eluting between 0.6 and 0.8 M KCl, were pooled and dialyzed against TCNa buffer (50 mM Tris, pH 7.5, 0.2 M NaCl, 2 mM CaCl₂).
The resulting enzyme was approximately purified 90-fold, with a recovery level of approximately 45%. Attempts to further purify N-proteinase using various chromatographic techniques were unsuccessful to the extent that poor enzyme recovery was observed. Consequently, a monoclonal antibody was developed to further purify the enzyme.

### 2. Production Of Monoclonal Antibody And Further Purification Of N-Proteinase

The enzymatic preparation after the heparin-Sepharose chromatography (*step IV,* above at *Section VII.A.1.*) was used for immunization of F1 mice (Balb/c x C57 Black/6, Studie Centrum voor Kernenergie, Mol, Belgium). Mice were intraperitoneally inoculated twice at 3-week intervals with 20 µg of antigen emulsified I Freund's adjuvant. Ten days after the second injection, the animals were boosted with 20 µg of antigen in saline and sacrificed 3 days later. Three-thousand (3000) hybridoma clones were screened for their ability to produce a monoclonal antibody able to immunoprecipitate N-proteinase activity in the presence of goat anti-mouse IgG coupled to agarose beads (Sigma). Only one hybridoma supernatant (clone 37D9) out of 3000 promoted significant and reproducible immunoprecipitation. The secreted monoclonal antibody was subclassed as an IgG1. After purification on a protein G column, 20 mg of antibody was coupled to 15 ml of Affi-Gel Hz hydrazide following instructions of the manufacturer (Bio-Rad) with an efficiency of 80% and used to further purify the enzyme as follows:

### Step V: Affinity Chromatography On Immobilized 37D9 Monoclonal

***Antibody.*** A maximum of 50 ml of the preparation at *step IV* was applied to an affinity column prepared as described below. After two successive washings in TCNa buffer and in 0.2 M ammonium acetate (NH₄Ac), the enzyme was eluted with 0.6 M NH₄Ac.

***Step VI: Second Chromatography On Heparin-Sepharose.*** The fractions collected in *step* V containing the enzymatic activity were pooled and loaded on a 0.5-ml heparin-Sepharose column. After washing in 0.8 M NH₄Ac, PCI-NP was eluted at 1.2 M NH₄Ac, PCI-NP was eluted at 1.2 M NH₄Ac and stored at -80°C.

### 3. Determination Of Amino Acid Sequence Encoding N-Proteinase

Amino-terminal sequence analysis of the intact protein and the peptides was performed on the model 476A protein sequencer (ABI, Foster City, CA) operating in the pulsed liquid mode with on-line phenylthiohydantoin analysis. The amino-terminal sequence analysis of the blotted PCI-NP was performed in a cross-flow reaction cartridge using modified run cycles. For sequence analysis of the peptides, trifluoroacetic acid-treated glass fiber disk was covered with polybrene before application of the sample. It was determined, using this method that the amino-terminal amino acid of PCI-NP was blocked and could not be subjected to an amino-terminal sequence analysis by Edman degradation. To overcome this problem, internal peptides of membrane-bound enzyme were produced and analyzed. More specifically, about 10 pmol (1 µg) of the blotted PCI-NP protein indicated that the protein was amino-terminally blocked, we cleaved the membrane-bound protein (30 µg) enzymatically to obtain sequence information for some internal peptide fragments. Endoproteinase Lys-C, which cleaves specifically at the carboxyl-terminal end of every lysyl residue, was chosen because the lysine content in PCI-NP (4%) seemed appropriate to obtain peptides of various lengths that could be easily resolved by reverse phase liquid chromatography analysis. A control digest was performed on a blank piece of PVDF (Coomassie stained but containing no protein) to identify peaks originating from background or enzyme autoproteolysis. After extraction, the peptides were separated on a reverse phase liquid chromatography column containing a mix of C₂/C₁₈ chains, and different fractions were collected. Several peptides were subjected to amino-terminal sequence analysis (3/4 of the material), but only two fractions, K1 and K3, contained a pure peptide that could be unambiguously sequenced up to the final lysyl residue. Three other fractions contained a mix of two or more fragments or had a very low initial sequence yield (< 1 pmol). As a final control, the remainder (1/4) of the sequenced fractions was subjected to matrix-assisted laser desorption/ionization mass analysis to verify the obtained sequences. For fraction K3, the calculated mass, 1633.7 Da, is in perfect agreement with the experimentally determined one, 1633.2 Da. Fraction K1 yielded no mass probably because there was too little material left.

The sequences for K1 and K3 were used to screen protein and nucleic acid data banks. No significant homology with known sequences was observed.

### 4. Cloning And Characterization Of Nucleic Acid Sequence Corresponding To N-Proteinase

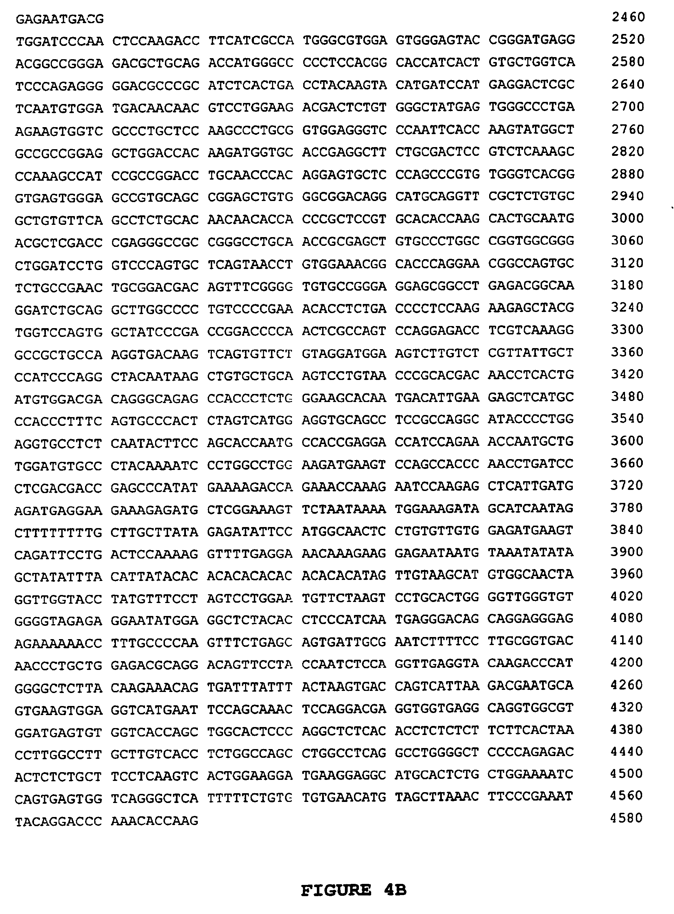

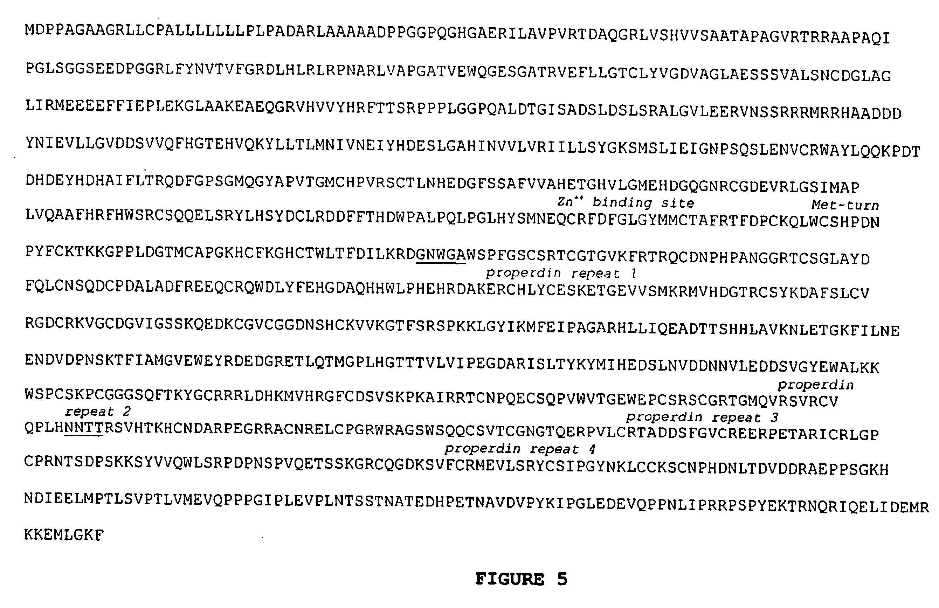

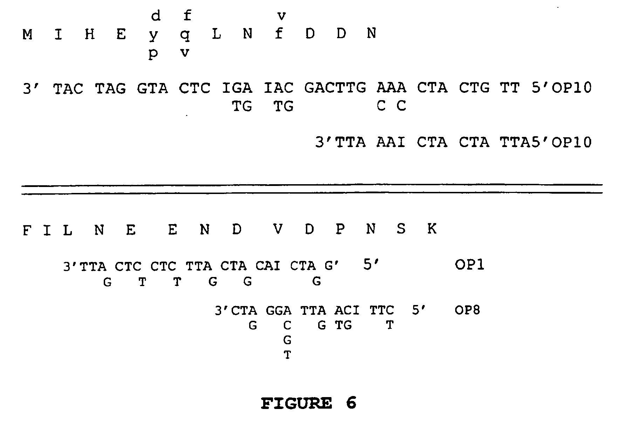

A bovine cDNA library was constructed using degenerated oligonucleotide primers, as set forth as OP 8 and OP 11 at FIGURE 6. These primers were designed from partial amino acid sequences reported in Colige et al., 1995, J. Biol. Chem. 270:16724-16730. The clones containing N-proteinase cDNA were identified after screening the bovine cDNA library with the longer degenerated oligonucleotides, OP 1 and OP 10, also as set forth in FIGURE 6. The bovine nucleotide sequence with the ATG start coding and the TAA stop codon (underlined) is set forth at FIGURES 4A and 4B. The deduced amino acid sequence, including the sequence's identifiable domain is set forth in FIGURE 5.

### 5. Isolation Of cDNAs For Human N-Proteinase

Total RNA was isolated from human skinfibroblast (CRL 1262) and reverse transcribed with oligo-dT and random primers to cDNA (Pharmacia cDNA synthesis kit). The cDNA then inserted into lambda phage vector ZAP II to make cDNA library. The cDNA library was screened for N-proteinase with ³²P-labeled bovine N-proteinase cDNA probe. Nine cDNA clones (size from 2 kb to 5 kb) were isolated from the library and sequenced with a fluorescent amplicycel sequencer (Perkin Elmer). According to the sequence, the full-length cDNA for human N-proteinase was generated.

### 6. Cloning Of Nucleic Acid Sequence Corresponding To Human N-Proteinase

Overlapping cDNA fragments were obtained by RT-PCR using oligonucleotide primers specific of human N-proteinase and RNA purified from four different strains of skin fibroblasts in culture. Sequences of the cDNA were determined using specific oligonucleotide primers and Thermo Sequenase radiolabeled cycle sequencing kit (Amersham).

### B. Example 2: Characterization of Polynucleotide Molecule Encoding Human N-Proteinase

The nucleotide sequence of human N-proteinase, both in its long and short form are set forth at Figures 1A-1C and Figure 2A. As set forth in FIGURE 3, N-proteinase and C-proteinase are similar in structure.

All references cited within the body of the instant specification are hereby incorporated by reference in their entirety.

## Claims

1. A polynucleotide sequence encoding N-proteinase, wherein said N-proteinase is:
(1) a protein encoded by the amino acid sequence set forth at Figure 1D deduced from the nucleic acid sequences set forth at Figures 1A-1C, a protein encoded by the amino acid sequences set forth at Figure 2B deduced from the nucleic acid sequence set forth at Figure 2A and the amino acid sequence encoded from the nucleic acid sequences set forth at Figures 4A-4B;
(2) a protein having N-proteinase activity wherein such protein is encoded by the amino acid sequences deduced from the nucleic acid sequences set forth at Figures 1A-1C, Figure 2A, and Figures 4A-4B, wherein one or more amino acids have been added, deleted, mutated, substituted or otherwise altered and the nucleotide sequence encoding said protein can hybridize to the nucleic acid sequence of Figures 1A-1C, Figure 2A and Figures 4A-4B under stringent hybridization conditions;
(3) the protein encoded by a naturally-occurring allele or homolog of the gene corresponding to the nucleic acid sequences set forth at Figures 1A-1C, Figure 2A, or Figures 4A-4B; or
(4) a fragment of (1), (2) or (3).

2. The polynucleotide sequence of claim 1, wherein said sequence is capable of hybridizing under stringent conditions to nucleic acid positions 3971-6692 of FIGURE 1B-1C.

3. The polynucleotide sequence of claim 1, wherein said sequence is capable of hybridizing under stringent conditions to nucleic acid positions 1-4580 of FIGURE 4A-4B.

4. A method for producing N-proteinase comprising:
a. transfecting or transforming a host cell with the polynucleotide sequence of claim 1;
b. culturing said host cell in appropriate culture media; and
c. isolating N-proteinase from said culture media.

5. A N-proteinase obtainable by a method comprising:
a. transfecting or transforming a host cell with a polynucleotide sequence encoding N-proteinase;
b. culturing said host cell in appropriate culture media; and
c. isolating N-proteinase from said culture media;
wherein said N-proteinase is:
(1) a protein encoded by the amino acid sequence set forth at Figure 1D deduced from the nucleic acid sequences set forth at Figures 1A-1C, a protein encoded by the amino acid sequences set forth at Figure 2B deduced from the nucleic acid sequence set forth at Figure 2A and the amino acid sequence encoded from the nucleic acid sequences set forth at Figures 4A-4B;
(2) a protein having N-proteinase activity wherein such protein is encoded by the amino acid sequences deduced from the nucleic acid sequences set forth at Figures 1A-1C, Figure 2A, and Figures 4A-4B, wherein one or more amino acids have been added, mutated or substituted and the nucleotide sequence encoding said protein can hybridize to the nucleic acid sequence of Figures 1A-1C, Figure 2A and Figures 4A-4B under stringent hybridization conditions; or
(3) the protein encoded by a naturally-occurring allele or homolog of the gene corresponding to the nucleic acid sequences set forth at Figures 1A-1C, Figure 2A, or Figures 4A-4B.

6. A host cell transformed or transfected with the polynucleotide sequence of claim 1.

7. Use of an N-proteinase according to claim 5 in a method for determining an antibody that inhibits the activity of N-proteinase.

8. An antibody to an epitope of an N-proteinase according to claim 5.

9. The antibody of claim 8, wherein said antibody is polyclonal, monoclonal, chimeric, single chain, a Fab fragment or a fragment produced by a Fab expression library.

10. The antibody according to claim 8 or claim 9, wherein the antibody is a neutralizing antibody.

11. An antibody according to claim 10 for use in diagnosis or therapy.

12. Use of an antibody according to claim 10 in the manufacture of a medicament for the treatment of fibrosis.

13. An antibody according to claim 8 or claim 9, wherein said antibody is a monoclonal antibody and is radioactively labelled.

14. An antibody according to claim 13 for use in diagnosis.

15. Use of an antibody according to claim 13 in the manufacture of a non-invasive diagnostic tool for imaging sites of collagen production.

16. The use according to claim 15, wherein the collagen production is associated with fibrosis or rheumatoid arthritis.

## Patentansprüche

1. N-Proteinase kodierende Polynukleotidsequenz, wobei die N-Proteinase
(1) ein Protein, das von der in Abbildung 1D dargelegten Aminosäuresequenz, die von den in Abbildungen 1A-1C dargelegten Nukleinsäuresequenzen abgeleitet ist, kodiert wird, ein Protein, das von den in Abbildung 2B dargelegten Aminosäuresequenzen, die von der in Abbildung 2A dargelegten Nukleinsäuresequenz abgeleitet sind, kodiert wird, und die von den in Abbildungen 4A-4B dargelegten Nukleinsäuresequenzen kodierte Aminosäuresequenz,
(2) ein Protein, das N-Proteinase-Aktivität aufweist, wobei ein derartiges Protein von den Aminosäuresequenzen kodiert wird, die von den in den Abbildungen 1A-1C, in Abbildung 2A und in den Abbildungen 4A-4B dargelegten Nukleinsäuresequenzen abgeleitet sind, wobei eine oder mehrere Aminosäuren hinzugefügt, deletiert, mutiert, substituiert oder auf andere Weise verändert wurden und die Nukleotidsequenz, die das Protein kodiert, unter stringenten Hybridisierungsbedingungen an die Nukleinsäuresequenz der Abbildungen 1A-1C, der Abbildung 2A und der Abbildungen 4A-4B hybridisieren kann,
(3) das von einem natürlich vorkommenden Allel oder Homolog des Gens, das den, in den Abbildungen 1A-1C, in Abbildung 2A oder in den Abbildungen 4A-4B dargelegten Nukleinsäuresequenzen entspricht, kodierte Protein, oder
(4) ein Fragment von (1), (2) oder (3), ist.

2. Polynukleotidsequenz nach Anspruch 1, wobei die Sequenz befähigt ist, unter stringenten Bedingungen an die Nukleinsäurepositionen 3971-6692 von Abbildung 1B-1C zu hybridisieren.

3. Polynukleotidsequenz nach Anspruch 1, wobei die Sequenz befähigt ist, unter stringenten Bedingungen an die Nukleinsäurepositionen 1-4580 von Abbildung 4A-4B zu hybridisieren.

4. Verfahren für die Herstellung von N-Proteinase, umfassend:
a. Transfizieren oder Transformieren einer Wirtszelle mit der Polynukleotidsequenz nach Anspruch 1,
b. Kultivieren der Wirtszelle in geeignetem Kulturmedium, und
c. Isolieren der N-Proteinase aus dem Kulturmedium.

5. N-Proteinase, erhältlich durch ein Verfahren, umfassend:
a. Transfizieren oder Transformieren einer Wirtszelle mit einer N-Proteinase kodierenden Polynukleotidsequenz,
b. Kultivieren der Wirtszelle in geeignetem Kulturmedium, und
c. Isolieren der N-Proteinase aus dem Kulturmedium,
wobei die N-Proteinase
(1) ein Protein, das von der in Abbildung 1D dargelegten Aminosäuresequenz, die von den in Abbildungen 1A-1C dargelegten Nukleinsäuresequenzen abgeleitet ist, kodiert wird, ein Protein, das von den in Abbildung 2B dargelegten Aminosäuresequenzen, die von der in Abbildung 2A dargelegten Nukleinsäuresequenz abgeleitet sind, kodiert wird, und die von den in Abbildungen 4A-4B dargelegten Nukleinsäuresequenzen kodierte Aminosäuresequenz,
(2) ein Protein, das N-Proteinase-Aktivität aufweist, wobei ein derartiges Protein von den Aminosäuresequenzen kodiert wird, die von den in den Abbildungen 1A-1C, in Abbildung 2A und in den Abbildungen 4A-4B dargelegten Nukleinsäuresequenzen abgeleitet sind, wobei eine oder mehrere Aminosäuren hinzugefügt, mutiert oder substituiert wurden und die Nukleotidsequenz, die das Protein kodiert, unter stringenten Hybridisierungsbedingungen an die Nukleinsäuresequenz der Abbildungen 1A-1C, der Abbildung 2A und der Abbildungen 4A-4B hybridisieren kann, oder
(3) das von einem natürlich vorkommenden Allel oder Homolog des Gens, das den, in den Abbildungen 1A-1C, in Abbildung 2A oder in den Abbildungen 4A-4B dargelegten Nukleinsäuresequenzen entspricht, kodierte Protein,
ist.

6. Wirtszelle, mit der Polynukleotidsequenz nach Anspruch 1 transformiert oder transfiziert.

7. Verwendung einer N-Proteinase nach Anspruch 5 in einem Verfahren für die Bestimmung eines Antikörpers, der die Aktivität von N-Proteinase hemmt.

8. Antikörper gegen ein Epitop einer N-Proteinase nach Anspruch 5.

9. Antikörper nach Anspruch 8, wobei der Antikörper polyklonal, monoklonal, chimär, einkettig, ein Fab-Fragment oder ein von einer Fab-Expressionsbibliothek hergestelltes Fragment, ist.

10. Antikörper nach Anspruch 8 oder Anspruch 9, wobei der Antikörper ein neutralisierender Antikörper ist.

11. Antikörper nach Anspruch 10 für die Verwendung in der Diagnose oder Therapie.

12. Verwendung eines Antikörpers nach Anspruch 10 in der Herstellung eines Medikaments für die Behandlung einer Fibrose.

13. Antikörper nach Anspruch 8 oder Anspruch 9, wobei der Antikörper ein monoklonaler Antikörper ist und radioaktiv markiert ist.

14. Antikörper nach Anspruch 13 für die Verwendung in der Diagnose.

15. Verwendung eines Antikörpers nach Anspruch 13 in der Herstellung eines nicht-invasiven diagnostischen Hilfsmittels für die Bildgebung von Orten der Kollagenproduktion.

16. Verwendung nach Anspruch 15, wobei die Kollagenproduktion mit einer Fibrose oder rheumatoider Arthritis assoziiert ist.

## Revendications

1. Séquence polynucléotidique codant pour une N-protéinase, ladite N-protéinase étant :
(1) une protéine codée par la séquence d'acides aminés présentée à la Figure 1D déduite des séquences d'acides nucléiques présentées aux Figures 1A-1C, une protéine codée par les séquences d'acides aminés présentées à la Figure 2B déduites de la séquence d'acides nucléiques présentée à la Figure 2A et de la séquence d'acides aminés codée à partir des séquences d'acides nucléiques présentées aux Figures 4A-4B ;
(2) une protéine ayant une activité de N-protéinase, ladite protéine étant codée par les séquences d'acides aminés déduites des séquences d'acides nucléiques présentées aux Figures 1A-1C, à la Figure 2A, et aux Figures 4A-4B, pour laquelle un ou plusieurs des acides aminés est/sont ajouté(s), délété(s), muté(s), substitué(s) ou modifié(s) autrement, et la séquence nucléotidique codant pour ladite protéine pouvant s'hybrider à la séquence d'acides nucléiques des Figures 1A-1C, de la Figure 2A, et des Figures 4A-4B dans des conditions d'hybridation stringentes ;
(3) la protéine codée par un allèle d'origine naturelle ou par un homologue du gène correspondant aux séquences d'acides nucléiques présentées aux Figures 1A-1C, à la Figure 2A, ou aux Figures 4A-4B ; ou
(4) un fragment de (1), (2) ou (3).

2. Séquence polynucléotidique selon la revendication 1, ladite séquence étant capable de s'hybrider dans des conditions stringentes aux positions 3971-6692 de l'acide nucléique des Figures 1B-1C.

3. Séquence polynucléotidique selon la revendication 1, ladite séquence étant capable de s'hybrider dans des conditions stringentes aux positions 1-4580 de l'acide nucléique des Figures 4A-4B.

4. Procédé de production d'une N-protéinase comprenant :
a. la transfection ou la transformation d'une cellule hôte par la séquence polynucléotidique selon la revendication 1 ;
b. la culture de ladite cellule hôte dans un milieu de culture approprié ; et
c. l'isolement de la N-protéinase à partir dudit milieu de culture.

5. Une N-protéinase pouvant être obtenue par un procédé comprenant :
a. la transfection ou la transformation d'une cellule hôte par une séquence polynucléotidique codant pour la N-protéinase ;
b. la culture de ladite cellule hôte dans un milieu de culture approprié ; et
c. l'isolement de la N-protéinase à partir dudit milieu de culture ;
dans lequel ladite N-protéinase est :
(1) une protéine codée par la séquence d'acides aminés présentée à la Figure 1D déduite des séquences d'acides nucléiques présentées aux Figures 1A-1C, une protéine codée par les séquences d'acides aminés présentées à la Figure 2B déduites de la séquence d'acides nucléiques présentée à la Figure 2A et de la séquence d'acides aminés codée à partir des séquences d'acides nucléiques présentées aux Figures 4A-4B ;
(2) une protéine ayant une activité de N-protéinase, ladite protéine étant codée par les séquences d'acides aminés déduites des séquences d'acides nucléiques présentées aux Figures 1A-1C, à la Figure 2A, et aux Figures 4A-4B, pour laquelle un ou plusieurs des acides aminés est/sont ajouté(s), muté(s) ou substitué(s), et la séquence nucléotidique codant pour ladite protéine pouvant s'hybrider à la séquence d'acides nucléiques des Figures 1A-1C, de la Figure 2A, et des Figures 4A-4B dans des conditions d'hybridation stringentes ; ou
(3) la protéine codée par un allèle d'origine naturelle ou par un homologue du gène correspondant aux séquences d'acides nucléiques présentées aux Figures 1A-1C, à la Figure 2A, ou aux Figures 4A-4B.

6. Cellule hôte transformée ou transfectée avec la séquence polynucléotidique selon la revendication 1.

7. Utilisation d'une N-protéinase selon la revendication 5 dans un procédé de détection d'un anticorps inhibant l'activité de la N-protéinase.

8. Anticorps dirigé contre un épitope d'une N-protéinase selon la revendication 5.

9. Anticorps selon la revendication 8, ledit anticorps étant un anticorps polyclonal, monoclonal, chimèrique, à chaîne unique, un fragment Fab ou un fragment produit par une banque d'expression de fragments Fab.

10. Anticorps selon la revendication 8 ou 9, ledit anticorps étant un anticorps neutralisant.

11. Anticorps selon la revendication 10, à usage diagnostique ou thérapeutique.

12. Utilisation d'un anticorps selon la revendication 10, dans la fabrication d'un médicament destiné au traitement de la fibrose.

13. Anticorps selon la revendication 8 ou 9, ledit anticorps étant un anticorps monoclonal marqué radioactivement.

14. Anticorps selon la revendication 13, à usage diagnostique.

15. Utilisation d'un anticorps selon la revendication 13, dans la fabrication d'un outil de diagnostic non invasif destiné à visualiser des sites de production de collagène.

16. Utilisation selon la revendication 15, où la production de collagène est associée à une fibrose ou à une polyarthrite rhumatoïde.
